# EUROPÄISCHE PATENTSCHRIFT

(11) **EP 3 501 730 B1**
(45) Veröffentlichungstag und Bekanntmachung des Hinweises auf die Patenterteilung: **12.06.2024**
(21) Anmeldenummer: 17210070.3
(22) Anmeldetag: 22.12.2017
(51) Int. Cl.: B23Q 17/09, A61C 13/00

(54) **DENTALFRÄSMASCHINE SOWIE VERFAHREN**
DENTAL MILL AND METHOD
MACHINE À FRAISER DENTAIRE AINSI QUE PROCÉDÉ

(43) Veröffentlichungstag der Anmeldung: 26.06.2019
(73) Patentinhaber: Ivoclar Vivadent AG, 9494 Schaan (LI)
(72) Erfinder: Huber, Martin, 5452 Pfarrwerfen (AT); Wörner, Alfons, 5500 Bischofshofen (AT)
(74) Vertreter: Baldus, Oliver

(56) Entgegenhaltungen:
- EP-A1- 0 474 588
- DE-U1-202015 100 312

## Beschreibung

Die Erfindung betrifft eine Dentalfräsmaschine, gemäß dem Oberbegriff von Anspruch 1 sowie ein Verfahren zum Betrieb einer solchen, gemäß dem Oberbegriff von Anspruch 11.

Fräsmaschinen weisen typischerweise mehrere Bewegungsachsen für einen Werkstück, und gegebenenfalls auch eine Bewegungsachse oder mehrere Bewegungsachsen für das Werkzeug auf. Dentalfräsmaschinen, bei denen eine 5/0-Verteilungen der Achsen vorliegt, die also fünf Bewegungsachsen für den Werkstück haben und keine für das Werkzeug, haben sich als besonders günstig herausgestellt.

Für die Genauigkeit der Bearbeitung ist es wesentlich, den Arbeitspunkt, also den Ort des Kontakts zwischen Werkstück und Werkzeug, zu kennen. Hierzu wird typischerweise eine Kalibrierung der Fräsmaschine vorgenommen.

Auch Werkzeuge, die mit vergleichsweise geringen Toleranzen hergestellt werden, unterliegen sie einem Verschleiß. Zudem ist die Einspannposition im Spannfutter nicht immer gleich, so dass eine Kalibrierung des eingespannten Werkzeugs unter Erfassung einer Referenzposition regelmäßig wiederkehrend durchgeführt werden muss.

Systeme zur Werkzeuglängenmessung sind Kernkomponenten von vollautomatisierten CNC-Maschinen. Herkömmliche Werkzeuglängenmesseinrichtungen sind jedoch für sehr kleine CNC-Maschinen nicht geeignet, da diese keine kosteneffektive Lösung darstellen.

Aus der EP 474 588 A1, die die Basis für den Oberbegriff der Ansprüche 1 und 11 bildet, ist eine Bearbeitungsvorrichtung mit Roboter bekannt, die sowohl für den werkzeuggeführten Betrieb als auch für den werkstückgeführten Betrieb des Roboters geeignet sein soll. Eine Anlage zwischen Werkzeug und einer Referenzfläche muss bei dieser Lösung manuell hergestellt werden.

Die DE 10 2013 100 155 A1 offenbart eine mechanische Schnittstelle für die Kopplung von einem Modul an einen Modulträger oder für die Kopplung von zwei Modulen umfassend eine erste Schnittstellenkomponente, eine zweite Schnittstellenkomponente, wobei die Schnittstellenkomponenten zueinander komplementär sind, ein Positionierungsmittel zur wiederholbaren Positionierung der zweiten Schnittstellenkomponente und der ersten Schnittstellenkomponente zueinander in einer vorbestimmten Position und ein Haltemittel zur Halterung der zweiten Schnittstellenkomponente an der ersten Schnittstellenkomponente, wobei die Schnittstelle ein Datenübertragungsmittel zur Übertragung von Daten zwischen der ersten Schnittstellenkomponente und der zweiten Schnittstellenkomponente und ein Leistungsübertragungsmittel zur Übertragung von elektrischer Leistung zwischen der ersten Schnittstellenkomponente und der zweiten Schnittstellenkomponente aufweist. Der Erfindung liegt somit die Aufgabe zugrunde, eine verbesserte Dentalfräsmaschine sowie ein verbessertes Verfahren zum Betreiben einer Dentalfräsmaschine bereitzustellen, welche es ermöglichen, die Dentalfräsmaschine automatisch zu kalibrieren und durch Messung einer Werkzeuglänge eines Fräswerkzeugs somit Herstellungstoleranzen des Fräswerkzeugs in die Kalibrierung der Dentalfräsmaschine miteinzubeziehen.

Die Aufgabe wird mit einer Dentalfräsmaschine mit den Merkmalen des Patentanspruchs 1 gelöst. Des Weiteren wird die Aufgabe mit einem Verfahren mit den Merkmalen des Patentanspruchs 11 gelöst.

### Offenbarung der Erfindung

Die vorliegende Erfindung schafft eine Dentalfräsmaschine mit einem Werkzeug, das in einem Spannfutter geführt ist, wobei das Spannfutter insbesondere drehbar, im Übrigen jedoch maschinenfest gelagert ist. Es ist ein Anlageelement an einem Werkstückarm, der gegenüber dem Werkzeug mindestens in Richtung der Spindelachse bewegbar gelagert ist, und eine Steuervorrichtung vorgesehen, die die Anlage zwischen dem Anlageelement und dem Fräswerkzeug, insbesondere an dessen Spitze, erkennt, indem diese aufeinander zu bewegt werden, bis sie aneinander anliegen und die Steuervorrichtung über das Abbremsen der (Relativ-)Bewegung beim Kontakt dieser die Anlage des Anlageelements an dem Werkzeug feststellt. Diese Position speichert sie als Referenzposition ab. Das Anlageelement ist an einem Werkstückarm angebracht, insbesondere von der Werkstückachse beabstandet.

Die genannte Bewegung kann entweder so erfolgen, dass das Werkzeug an einer stationären Antriebsspindel eingespannt ist, und der Werkstückarm mit dem Anlageelement sich bewegt, oder so, dass der Werkstückarm stationär ist und das Werkzeug sich zu ihm bewegt. Bevorzugt ist der Antriebsmotor für das Werkzeug ausgeschaltet. Es ist aber auch möglich, dass das Werkzeug sich langsam dreht.

Die Erfindung schafft des Weiteren ein Verfahren zum Betreiben einer Dentalfräsmaschine. Das Verfahren umfasst ein Bereitstellen eines Werkzeugs, das in einem Spannfutter geführt ist, wobei das Spannfutter insbesondere drehbar, im Übrigen jedoch maschinenfest gelagert ist und wobei das Fräswerkzeug in das Spannfutter eingespannt ist. Ein an dem Werkzeugarm angebrachtes Anlageelement ist gegenüber dem Werkzeug mindestens in Richtung der Spindelachse bewegbar gelagert.

Das Verfahren umfasst überdies ein Bereitstellen einer Steuervorrichtung, die einen Kontakt zwischen dem Werkzeug und dem Anlageelement erfasst. Hierzu wird der Motorstrom für den Antrieb des Werkstückarms bei Annäherung an das Werkzeug reduziert. Der Werkstückarm bewegt sich dadurch mit geringerer Kraft und vermeidet dadurch eine Beschädigung des Werkzeugs.

Bei Anlage, also beim Kontakt, wird die Bewegung abgebremst. Dies erfasst ein Encoder, der mit der Achse des Antriebsmotor mechanisch gekoppelt ist. Dessen Ausgangssignal wird der Steuervorrichtung zugeleitet. Steuervorrichtung und Anlageelement bilden insofern einen Sensor.

Eine Idee der vorliegenden Erfindung ist es, durch Vorsehen des Sensors eine genaue Vermessung der Länge des Fräswerkzeugs der Dentalfräsmaschine bereitzustellen. Insbesondere vorteilhaft ist die Tatsache, dass der Sensor in der Dentalfräsmaschine integriert ist und über den Sensor selbst in Verbindung mit einer entsprechenden Auswertevorrichtung die Längendaten betreffend die Länge des Fräswerkzeugs gewonnen werden können. Somit kann auf das Vorsehen einer externen Messvorrichtung, wie beispielsweise einer optischen Messvorrichtung, verzichtet werden.

Damit ergibt sich die Möglichkeit, eine genaue Referenzposition für die Bearbeitung des Werkstücks in der Dental-Fräsmaschine bereitzustellen, und zwar automatisch bei jedem Werkzeugwechsel, indem das erfindungsgemäße Verfahren durchgeführt wird. Die beim Kontakt ermittelte Position entspricht genau der Stelle, an welcher das Werkzeug - später - das Werkstück bearbeitet.

Der Kontakt mit dem Anlageelement kann genau in der Spindelachse erfolgen, also zentral, oder auch leicht, also um weniger als der Werkzeugdurchmesser seitlich versetzt. Es ist auch möglich, 2 Referenzpunkte zu bestimmen, also einen zentralen und einen seitlich versetzten. Damit läßt sich nicht nur die Länge, sondern auch die Form des Werkzeugs an dessen Spitze bestimmen und erkennen.

Dies ist besonders bei einem teilabgenutzten Werkzeug wichtig.

Die erfindungsgemäße Lösung erlaubt es sowohl, ein neues Werkzeug einzumessen, und zwar sowohl dessen absolute Länge ab dem Schaft als auch dessen Einspannlänge, also die aktuelle Referenzposition in dem eingespannten Zustand, aber auch die gleichen Feststellungen bei einem bereits benutzten und wieder eingespannten Werkzeug.

Bevorzugt wird die erfindungsgemäße Lösung bei jedem Werkzeugwechsel vorgenommen.

Vorteilhafte Ausführungsformen und Weiterbildungen ergeben sich aus den Unteransprüchen sowie aus der Beschreibung unter Bezugnahme auf die Figuren.

Gemäß einer weiteren bevorzugten Weiterbildung ist vorgesehen, dass eine Fräswerkzeug-Erkennungsvorrichtung vorgesehen ist, die das betreffende Fräswerkzeug anhand eines Codes, insbesondere eines optischen zweidimensionalen Codes, insbesondere vorzugsweise eines Data-Matrix-Codes, erfasst, wobei das Fräswerkzeug vorvermessen und seine Länge abgespeichert ist, insbesondere - auch - in der Steuervorrichtung. Somit kann das Fräswerkzeug sicher und zuverlässig erfasst werden.

Gemäß einer weiteren bevorzugten Weiterbildung ist vorgesehen, dass eine Temperaturerfassungsvorrichtung vorgesehen ist und dass die Steuervorrichtung einen Speicher für eine Dehnungstabelle aufweist, die die experimentell bestimmte Längung des Fräswerkzeugs basierend auf einer Temperaturänderung abspeichert und basierend auf dieser Längenänderung die exakte Position der Spitze des Fräswerkzeugs angibt. Somit ist die Position der Spitze des Fräswerkzeugs in vorteilhafter Weise bekannt und es muss somit lediglich mit dem Encoder, der die Bewegung des Anlageelements erfasst, die Position der Spitze des Werkzeugs erfasst werden, um die exakte Länge des Fräswerkzeugs zu vermessen.

Gemäß einer weiteren bevorzugten Weiterbildung ist vorgesehen, dass Düsen dazu ausgebildet sind, zur Nassbearbeitung eines Werkstücks einen Flüssigkeitsstrahl auf das Fräswerkzeug zu richten, und wobei die Düsen dazu ausgebildet sind, Druckluft an eine Umgebung abzugeben. Somit sind die Düsen in vorteilhafter Weise für eine Mehrzahl von Zwecken einsetzbar.

### Kurze Beschreibung der Zeichnungen

Die beiliegenden Zeichnungen sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung.

Andere Ausführungsformen und viele der genannten Vorteile ergeben sich im Hinblick auf die Zeichnungen. Die dargestellten Elemente der Zeichnungen sind nicht notwendigerweise maßstabsgetreu zueinander gezeigt.

Es zeigen:
- Fig. 1: eine perspektivische schematische Darstellung der Dentalfräsmaschine gemäß einer bevorzugten Ausführungsform der Erfindung, im Bearbeitungszustand;
- Fig. 2: eine Darstellung der in Fig. 1 gezeigten Dentalfräsmaschine, jedoch ohne Werkstück und unter Darstellung des erfindungsgemäßen Anlageelements; und
- Fig. 3: eine Darstellung des erfindungsgemäßen Anlageelements zum Erfassen einer Länge und/Lage des Fräswerkzeugs gemäß der bevorzugten Ausführungsform der Erfindung.

In den Figuren der Zeichnungen bezeichnen gleiche Bezugszeichen gleiche oder funktionsgleiche Elemente, Bauteile oder Komponenten, soweit nichts Gegenteiliges angegeben ist.

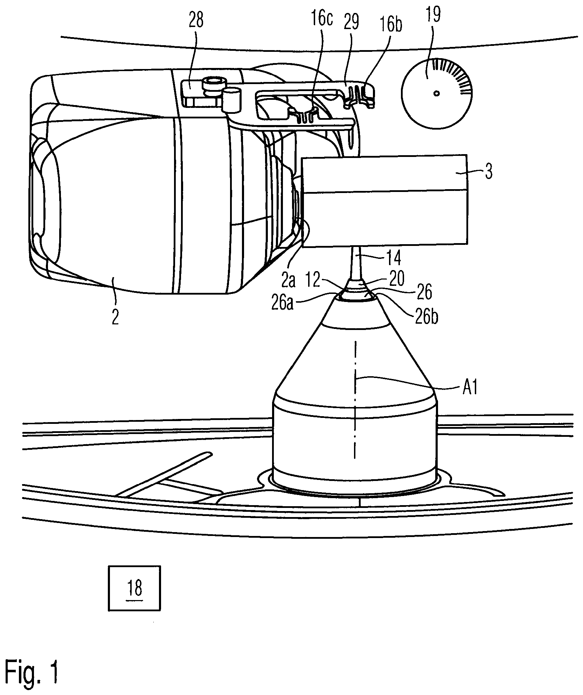

Fig. 1 zeigt eine schematische Darstellung der Dentalfräsmaschine gemäß einer bevorzugten Ausführungsform der Erfindung.

Die Dentalfräsmaschine 1 weist ein Werkzeug 14 auf, die in einem Spannfutter 12 geführt ist. Die Dentalfräsmaschine 1 weist überdies einen Roboterarm, hier Werkstückarm 2 genannt, auf, welcher mit einem Spannfutter 2a versehen ist, in welches ein Werkstück 3 mit einer entsprechenden (in Fig. 1 nicht gezeigten) Werkstückhalterung einsetzbar ist.

Das Spannfutter 12 ist insbesondere drehbar, im Übrigen jedoch maschinenfest gelagert.

Das Fräswerkzeug 14 ist in dem Spannfutter 12 mit seinem nicht ersichtlichen Schaft eingespannt gehalten.

Der Werkstückarm 2 ist gegenüber dem Werkzeug 14 mindestens in Richtung einer Spindelachse A1 bewegbar gelagert. Des Weiteren weist die Dentalfräsmaschine 1 eine Steuervorrichtung 18 auf. Die Steuervorrichtung 18 ist dazu ausgebildet, über einen Encoder 19, der mit einem nicht dargestellten Antriebsmotor des Werkstückarms 2 gekoppelt ist, die Relativposition des Werkstückarms 2 zum Fräswerkzeug 14 zu erfassen.

Das Fräswerkzeug 14 weist einen Ring 20 ausgebildet. Der Ring 20 ist von der Spitze 14a des Fräswerkzeugs 14 beabstandet angeordnet und dient als Anschlag bei Einspannen in das Spannfutter 12.

Der Ring 20 ist auf das Fräswerkzeug 14 am Übergang zu dessen Schaft 23 aufgebracht.

Der Schaft 23 weist eine plane Oberfläche mit einer Genauigkeit von insbesondere 15 µm auf, bezogen auf den Abstand D zur Spitze des Fräswerkzeugs. Die Spitze 14a des Fräswerkzeugs ist vorzugsweise mit einer Diamant-Bestückung versehen. Alternativ können andere Spitzen 14a, beispielsweise metallisch realisierte Spitzen, vorgesehen sein.

Eine Fräswerkzeug-Erkennungsvorrichtung 22 ist vorgesehen. Die Fräswerkzeug-Erkennungsvorrichtung 22, beispielsweise eine Kamera, erfasst das betreffende Fräswerkzeug 14 anhand eines Codes, insbesondere eines optischen zweidimensionalen Codes, insbesondere vorzugsweise eines Data-Matrix-Codes. Das Fräswerkzeug ist vorvermessen und sein Abstand D zwischen Ring 20 und Spitze 14a des Fräswerkzeugs 14 abgespeichert, insbesondere auch in der Steuervorrichtung 18. Des Weiteren weist die Dentalfräsmaschine 1 eine Temperaturerfassungsvorrichtung 24 auf. Die Steuervorrichtung 18 weist einen Speicher für eine Dehnungstabelle auf, die die experimentell bestimmte Längung des Fräswerkzeugs basierend auf einer Temperaturänderung abspeichert und basierend auf dieser Längenänderung die exakte Position der Spitze des Fräswerkzeugs angibt. Der Ring 20 weist insbesondere ein Nut auf, über welche das Fräswerkzeug 10 mittels eines Werkzeug-Wechselarms, beispielsweise des Greifhalters 29 am Werkstückarm 2, fassbar und aus dem Spannfutter entnehmbar ist. In dem dort gelagerten Zustand kann das Werkzeug vor die Kamera 22 geführt werden, um vorstehend beschriebene Fräswerkzeug-Erkennung vorzunehmen.

Die im Wesentlichen ringförmig ausgebildete Fläche 26, die das Spannfutter umgibt, weist überdies eine Mehrzahl von Düsen 26a auf. Die Düsen 26a weisen zum einen die Funktion auf, einen Flüssigkeitsstrahl auf das Fräswerkzeug 14 zu richten, wodurch eine Nassbearbeitung eines Werkstücks durch das Fräswerkzeug 14 ermöglicht wird. Darüber hinaus weisen die Düsen 26a die zusätzliche Funktion auf, dass durch die Düsen 26a Druckluft an eine Umgebung abgebbar ist. Durch einen Ausstoß von Druckluft durch die Düsen 26a kann eine Reinigung des Rings 20 bzw. des Fräswerkzeugs 14 von Verunreinigungen durchgeführt werden.

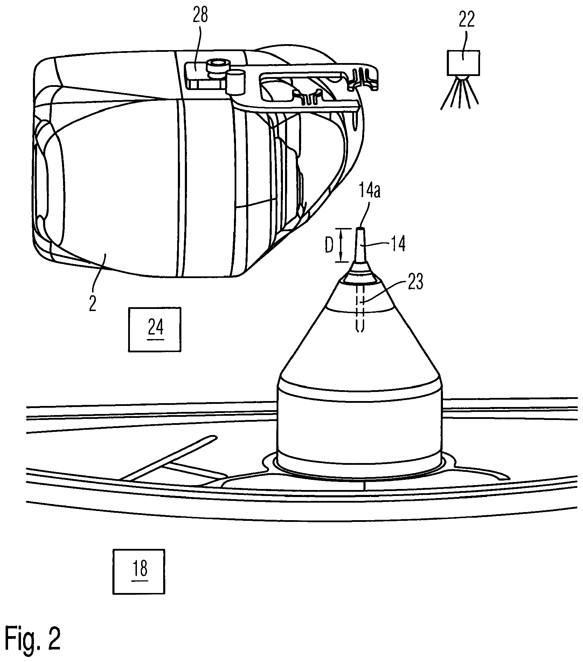

Fig. 1 und 2 zeigen eine schematische Darstellung eines am Werkstückarm 2 angebrachten Anlageelements 28 zum Erfassen einer Länge des Fräswerkzeugs gemäß der bevorzugten Ausführungsform der Erfindung. Das Anlageelement 28 ist als im Wesentlichen plan ausgebildetes Metallblech ausgebildet und ist weiter unten näher beschrieben. Es ist dem Greifhalter 29 für ein Fräswerkzeug 14 benachbart oder gemeinsam mit diesem befestigt.

Der Greifhalter 29 für das Fräswerkzeug 14 weist ein erstes Halterungselement 16b und ein zweites Halterungselement 16c auf. Das erste Halterungselement und das zweite Halterungselement sind derart ausgebildet, dass das jeweilige Fräswerkzeug 14 im Bereich einer der Nuten des Rings 20 durch das erste Halterungselement 16b oder das zweite Halterungselement 16c greifbar ist, indem das erste Halterungselement 16b oder das zweite Halterungselement 16c mit der Nut des Rings 20 des Fräswerkzeugs 14 in Eingriff bringbar ist.

Das erste Halterungselement 16b und das zweite Halterungselement 16c sind derart versetzt zueinander an dem Werkstückarm 2 angeordnet, dass während ein Fräswerkzeug 14 beispielsweise in dem Halterungselement 16c eingefügt ist, durch das Halterungselement 16b ein weiteres Fräswerkzeug, welches in dem Spannfutter 12 angeordnet ist, durch das erste Halterungselement 16b aus dem Spannfutter 12 herausnehmbar ist und in einem nachfolgenden Arbeitsschritt das in dem Halterungselement 16c eingesetzte Fräswerkzeug 14 in das dann unbestückte Spannfutter 12 einfügbar ist.

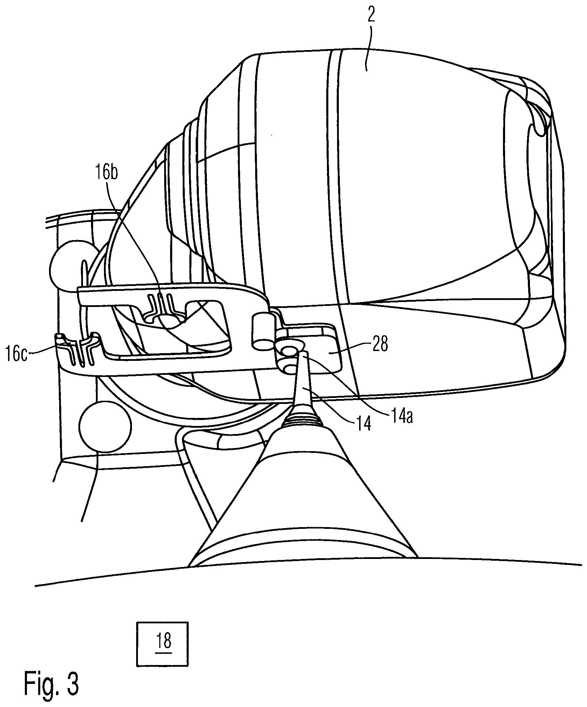

Das Anlageelement 28 ist in der in Fig. 1 dargestellten Position oben an dem Werkstückarm 2 . Der Werkstückarm 2 ist im Raum in fünf Achsen verfahrbar, unter anderem auch so drehbar, dass das Anlageelement 28 sich unten befindet kann (Fig. 3).

Das Anlageelement ist seitlich an dem Werkstück am 2 angebracht. Aus Fig. 2 ist ein Zustand der erfindungsgemäßen Dentalfräsmaschine ersichtlich, welchem eine Referenz-Positionsbestimmung möglich ist.

In diesem Zustand ist es nicht erforderlich, dass der Werkstückarm 2 ein Werkstück 3 trägt.

Die Referenzpunktbestimmung bezieht sich auf die Feststellung der tatsächlichen Position der Spitze 14a des Werkzeugs 14 bezogen auf den Werkstückarm, in welchen später dann das Werkstück 3 eingespannt ist.

Auch wenn hier der Werkstückarm 2 als beweglich und das Werkzeug 14 mit seinem Spindelmotor als raumfest dargestellt ist, versteht es sich, dass insofern auch eine kinematische Vertauschung möglich ist, ohne den Bereich der Erfindung zu verlassen.

Die Referenz-Positionsbestimmung erfolgt dem Grunde nach so, dass der Werkstückarm 2 mit nach unten weisendem Anlageelement 28 in Richtung des Pfeils 31 abgesenkt wird, bis das Anlageelement 28 mit dem Werkzeug 14 Kontakt hat.

Die Kontaktposition ist auch aus Fig. 3 ersichtlich. Das Anlageelement 28 besteht bevorzugt aus gehärtetem Stahlblech und ist mit hoher Präzision an dem Werkstückarm 2 montiert. Die Spitze 14a des Werkzeugs 14 trifft an einer planen Stelle auf das Anlageelement 28.

Es versteht sich, dass zusätzlich bei Bedarf auch eine zweite Referenzerfassung vorgenommen werden kann, indem durch Schrägstellung des Anlageelements 28 der Verschleiß des Werkzeugs 14 an dessen Spitze erfasst wird.

Die Anlageerfassung erfolgt bevorzugt über einen Schlupfsensor. Hierzu wird der Encoder 19 eingesetzt, der mit dem Antriebsmotor für die Vertikalbewegung des Werkstückarms 2 gekoppelt ist.

Der Encoder 19 gibt Signale zur Bewegung des Werkstückarm 2 in Richtung Werkzeugspitze 14a ab.

Für die Referenz-Positionserfassung wird nun der Antriebsmotor mit einem geringeren

Antriebsstrom als üblich versorgt. Dies ermöglicht eine weiche Bewegung nach unten. Der Encoder 19 erkennt die lineare Bewegung und gibt die entsprechenden Signale an die Steuervorrichtung 18 ab.

Wen nun das Anlageelement 28 an dem Werkzeug 14 anliegt, gibt der Encoder 19 nur diejenigen Signale ab, die einer Bewegung des Werkstückarms 2 nach unten entsprechen. Der Antriebsmotor versucht, das Anlageelement 28 nach unten zu verfahren, was jedoch nicht gelingt, da es an dem Werkzeug 14 anliegt.

Dementsprechend entsteht ein Schlupf zwischen dem Steuersignal für den Antriebsmotor, das ebenfalls von der Steuervorrichtung 18 abgegeben wird, und dem der tatsächlichen Bewegung, entsprechend den Signalen des Encoders 19.

Das erste Mal, dass ein Antriebsschritt übersprungen wird, also der erste Schlupf, wird nun erfasst und als Indiz für die Anlage von der Steuervorrichtung gewertet und entsprechend signalisiert.

In vorteilhafter Weiterbildung wird der Antriebsmotor im Normalbetrieb im Open-Loop-Betrieb betrieben, was Geschwindigkeitsvorteile mit sich bringt. Bei der Referenzermittlungen wird er hingegen bevorzugt im closed-loop-Betrieb betrieben.

Die erfasste Referenzposition wird auch abgespeichert und läßt sich für die Kalibrierung des Dentalfräsmaschine auf das je vermessene Werkzeug 14 verwenden.

Über der werkzeugspezifischen Code lassen sich auch die Betriebsstunden des Werkzeugs von der Steuervorrichtung 18 erfassen. Wenn das Werkzeug 14 nach nur wenigen Betriebsstunden erneut eingesetzt wird, ist ggf. eine Neuvermessung, also eine neue Referenzpunktbestimmung nicht erforderlich. Diese kann aber natürlich bei Bedarf vorgenommen werden.

Beispielsweise ist eine Dimensionierung bzw. geometrische oder materialtechnische Ausbildung des Positionssensors variierbar bzw. an jeweilige bauliche Anforderungen der Dentalfräsmaschine anpassbar.

## Patentansprüche

1. Dentalfräsmaschine (1) mit einem Werkzeug (14), das in einem Spannfutter (12) eingespannt an einer Spindel geführt ist, wobei das Spannfutter (12) insbesondere drehbar, im Übrigen jedoch maschinenfest gelagert ist, mit einem Werkstückarm (2), der gegenüber dem Werkzeug (14) mindestens in Richtung der Spindelachse bewegbar gelagert ist, und mit einer Steuervorrichtung (18), mit welcher dieser, insbesondere mittels eines Antriebsmotors des Werkstückarmes (2), relativ zum Werkstück (3) verfahrbar ist, **dadurch gekennzeichnet**, das der Werkstückarm (2) ein Anlageelement (28) aufweist, insbesondere von der Werkstückachse beabstandet, wobei mit der Steuervorrichtung (18) das Werkzeug (14) und das Anlageelement (28) aufeinander zu bewegbar sind, bis sie aneinander anliegen, und mit der Steuervorrichtung (18) die Anlage des Anlageelements (28) an dem Werkzeug (14), insbesondere an dessen Spitze (14a), erfassbar ist, insbesondere über das Abbremsen der (Relativ-) Bewegung von Werkzeug (14) und Anlageelement (28) aufeinander zu beim Kontakt von Werkzeug (14) und Anlageelement (28), und diese Position als Referenzposition signalisierbar und insbesondere abspeicherbar ist.

2. Dentalfräsmschine (1) nach Anspruch 1, **dadurch gekennzeichnet, dass** der Antriebsmotor als Schrittmotor ausgebildet und mit einem Encoder (19) verbunden ist, wobei ein durch den Encoder (19) festgestellter Schrittverlust des Schrittmotors als Anlage des Anlageelements (28) an dem Werkzeug (14), insbesondere dessen Spitze (14a), signalisiert oder festgestellt wird.

3. Dentalfräsmaschine (1) nach einer der vorhergehenden Ansprüche, **dadurch gekennzeichnet, dass** der Antriebsmotor für die Feststellung der Referenzposition an der Spitze (14a) des Werkzeugs (14) mit einem gegenüber einem Nenn-Antriebsstrom verminderten Antriebsstrom betrieben wird.

4. Dentalfräsmaschine (1) nach einem der vorhergehenden Ansprüche, **dadurch gekennzeichnet, dass** das Anlageelement (28) seitlich an dem Werkstückarm (2), von der Achse des Werkstücks (3) um einen vorgegebenen Betrag beabstandet und insbesondere auch von dem Werkstück (3) beabstandet, angebracht ist.

5. Dentalfräsmaschine (1) nach einem der vorhergehenden Ansprüche, **dadurch gekennzeichnet, dass** mit der Steuervorrichtung (18) die Relativbewegung des Anlageelements (28) und der Spitze (14a) des Werkzeugs (14) als lineare, translatorische Bewegung zum Werkzeug (14) hin und von diesem weg steuerbar ist.

6. Dentalfräsmaschine(1) nach einem der vorhergehenden Ansprüche, **dadurch gekennzeichnet, dass** mit der Steuervorrichtung (18) der Werkstückarm (2) mit dem Anlageelement (28) zur Spitze (14a) des Werkzeugs (14) dergestalt steuerbar ist, dass das Anlageelement (28) die Spitze (14a) des Werkzeugs (14) immer an der gleichen Stelle kontaktiert, auch beim Werkzeugwechsel.

7. Dentalfräsmaschine (1) nach einem der vorhergehenden Ansprüche, **dadurch gekennzeichnet, dass** die Dentalfräsmaschine (1) als 5/0 Werkzeugmaschine ausgebildet ist, also mit fünf Bewegungsachsen des Werkstückarms (2).

8. Dentalfräsmaschine (1) nach Anspruch 2, **dadurch gekennzeichnet, dass** die Steuervorrichtung (18) für die Referenzpunktermittlung den Encoder (19) des Antriebsmotors des Werkzeugarms (2) von Open-Loop auf Closed-Loop umschaltet.

9. Dentalfräsmaschine (1) nach Anspruch 2, **dadurch gekennzeichnet, dass** die Kombination aus Steuervorrichtung (18) und Encoder (19) als Schlupfsensor ausgebildet ist, über welchen die Steuervorrichtung (18) beim Auftreten von Schlupf bei der Bewegung des Werkstückarms (2) zur Werkzeugspitze hin die Anlage des Anlageelements (28) an der Werkzeugspitze feststellt oder signalisiert.

10. Dentalfräsmaschine (1) nach einem der vorhergehenden Ansprüche, **dadurch gekennzeichnet, dass** das Fräswerkzeug (14) einen Code aufweist und eine Fräswerkzeug-Erkennungsvorrichtung (22) das Fräswerkzeug (14) anhand dieses Codes werkzeugspezifisch erfasst und die Erfassung in der Steuervorrichtung (18) abgespeichert wird.

11. Verfahren zur Ermittlung der Position Spitze (14a) eines Werkzeugs (14) einer Dentalfräsmaschine (1) für die Festlegung eines Referenzpunktes, wobei das Werkzeug (14) in einem Spannfutter (12) eingespannt ist und wobei ein Werkstückarm (2) ein Anlageelement (28) aufweist, an einer Stelle, die von dem Werkstück (3), insbesondere dessen Achse, um einen vorgegebenen Wert beabstandet ist, **dadurch gekennzeichnet, dass** für die Referenzpunktermittlung der Werkstückarm (2) mit dem Anlageelement (28) von einem Antriebsmotor für den Werkstückarm (2) translatorisch relativ zu der Spitze (14a) des Werkzeugs (14) bewegt wird, wobei die Bewegung des Werkstückarms (2) über einen Encoder (19), der mit dem Antriebsmotor gekoppelt ist, erfasst wird, und dass der Encoder (19) zusammen mit der Steuervorrichtung (18) als Schlupfsensor wirkt, der erkennt, wenn durch die Anlage des Anlageelements (28) an der Spitze (14a) des Werkzeugs (14) Antriebsschlupf entsteht, und dass das erstmalige Auftreten von Antriebsschlupf als Anzeichen für die Anlage und damit von der Steuervorrichtung (18) als Referenzposition gewertet wird.

12. Verfahren nach Anspruch 11, **dadurch gekennzeichnet, dass** der Werkstückarm (2) mit dem Anlageelement (28) zu der Spitze (14a) des Werkzeugs (14), das in einer fest angebrachten Werkzeugspindel eingespannt ist, über einen als Schrittmotor ausgebildeten Antriebsmotor bewegt wird und ein Schrittverlust des Schrittmotors für die Schlupferkennung verwendet wird.

## Claims

1. A dental milling machine (1) comprising a tool (14) which is clamped in a chuck (12) and guided at a spindle, wherein the chuck (12) is mounted particularly rotatably, but fixed to the machine apart from that, comprising a workpiece arm (2) which is mounted to be movable relative to the tool (14) at least in the direction of the spindle axis, and comprising a control device (18) which is used to move the latter, in particular by means of a drive motor of the workpiece arm (2), relative to the workpiece (3), **characterized in that** the workpiece arm (2) comprises a contact element (28), in particular spaced apart from the workpiece axis, wherein the control device (18) is used to move the tool (14) and the contact element (28) towards each other until they contact each other, and that the control device (18) is used to detect contact of the contact element (28) with the tool (14), in particular with its tip (14a), in particular via deceleration of the (relative) movement of tool (14) and contact element (28) towards each other upon contact of tool (14) and contact element (28), and that this position can be signaled and in particular stored as a reference position.

2. The dental milling machine (1) as claimed in claim 1, **characterized in that** the drive motor is configured as a stepping motor and connected to an encoder (19) wherein a step loss of the stepping motor determined by the encoder (19) is signalized or determined as a contact of the contact element (28) with the tool (14), in particular its tip (14a).

3. The dental milling machine (1) as claimed in any of the preceding claims, **characterized in that** the drive motor for determining the reference position at the tip (14a) of the tool (14) is operated with a driving current which is reduced compared to a nominal driving current.

4. The dental milling machine (1) as claimed in any of the preceding claims, **characterized in that** the contact element (28) is disposed on the side of the workpiece arm (2), spaced apart from the axis of the workpiece (3) by a given amount and in particular also spaced apart from the workpiece (3).

5. The dental milling machine (1) as claimed in any of the preceding claims, **characterized in that** the control device (18) is used to control the relative movement of the contact element (28) and the tip (14a) of the tool (14) as a linear, translational movement towards the tool (14) and away from it.

6. The dental milling machine (1) as claimed in any of the preceding claims, **characterized in that** the control device (18) is used to control the workpiece arm (2) with the contact element (28) to the tip (14a) of the tool (14) in such a way that the contact element (28) contacts the tip (14a) of the tool (14) always at the same place, even during tool change.

7. The dental milling machine (1) as claimed in any of the preceding claims, **characterized in that** the dental milling machine (1) is configured as a 5/0-machine tool, i.e. comprising five axes of movement of the workpiece arm (2).

8. The dental milling machine (1) as claimed in claim 2, **characterized in that** the control device (18) for reference point determination switches the encoder (19) of the drive motor of the workpiece arm (2) from open loop to closed loop.

9. The dental milling machine (1) as claimed in claim 2, **characterized in that** the combination of control device (18) and encoder (19) is configured as a slip sensor via which the control device (18) determines or signalizes contact of the contact element (28) with the tool tip upon occurrence of slip during movement of the workpiece arm (2) towards the tool tip.

10. The dental milling machine (1) as claimed in any of the preceding claims, **characterized in that** the milling tool (14) comprises a code and a milling tool detection device (22) performs a tool-specific detection of the milling tool (14) based on this code and that the detection is stored in the control device (18).

11. A method of determining the position of the tip (14a) of a tool (14) of a dental milling machine (1) for determining a reference point, wherein the tool (14) is clamped in a chuck (12) and wherein a workpiece arm (2) comprises a contact element (28) at a place spaced apart from the workpiece (3), in particular its axis, by a predetermined value, **characterized in that** for determining the reference point the workpiece arm (2) with the contact element (28) is moved by a drive motor for the workpiece arm (2) translationally relative to the tip (14a) of the tool (14), wherein the movement of the workpiece arm (2) is detected by an encoder (19) which is coupled to the drive motor, and that the encoder (19), along with the control device (18), acts as a slip sensor which detects drive slip caused by contact of the contact element (28) at the tip (14a) of the tool (14), and that first-time occurrence of drive slip is considered an indication for contact and thus as a reference position by the control device (18) .

12. The method as claimed in claim 11, **characterized in that** the workpiece arm (2) with the contact element (28) is moved towards the tip (14a) of the tool (14), which is clamped in a fixedly arranged tool spindle, by a drive motor configured as a stepping motor, and that a step loss of the stepping motor is used to detect slip.

## Revendications

1. Fraiseuse dentaire (1) comprenant un outil (14) qui est guidé serré dans un mandrin de serrage (12) sur une broche, où le mandrin de serrage (12) est en particulier rotatif, mais par ailleurs est monté de manière fixe sur la machine, un bras porte-pièce (2) qui est monté mobile par rapport à l'outil (14) au moins dans la direction de l'axe de la broche, et un dispositif de commande (18) avec lequel celui-ci peut être déplacé par rapport à l'outil (14), en particulier au moyen d'un moteur d'entraînement du bras porte-pièce (2), **caractérisée en ce que** le bras porte-pièce (2) est monté mobile par rapport à la pièce à usiner (3), le bras (2) de la pièce à usiner présente un élément d'appui (28), en particulier à distance de l'axe de la pièce à usiner, où, avec le dispositif de commande (18), l'outil (14) et l'élément d'appui (28) peuvent être déplacés l'un vers l'autre jusqu'à ce qu'ils soient en appui l'un contre l'autre, et le dispositif de commande (18) permet de détecter l'appui de l'élément d'appui (28) sur l'outil (14), en particulier sur sa pointe (14a), en particulier par le biais du freinage du mouvement (relatif) de l'outil (14) et de l'élément d'appui (28) l'un vers l'autre lors du contact entre l'outil (14) et l'élément d'appui (28), et cette position peut être signalée comme position de référence et, en particulier, être mémorisée.

2. Fraiseuse dentaire (1) selon la revendication 1, **caractérisée en ce que** le moteur d'entraînement est conçu comme un moteur pas à pas et est relié à un encodeur (19), où une perte de pas du moteur pas à pas constatée par l'encodeur (19) est signalée ou constatée en tant qu'appui de l'élément d'appui (28) sur l'outil (14), en particulier sa pointe (14a).

3. Fraiseuse dentaire (1) selon l'une des revendications précédentes, **caractérisée en ce que** le moteur d'entraînement pour la détermination de la position de référence à la pointe (14a) de l'outil (14) fonctionne avec un courant d'entraînement réduit par rapport à un courant d'entraînement nominal.

4. Fraiseuse dentaire (1) selon l'une des revendications précédentes, **caractérisée en ce que** l'élément d'appui (28) est monté latéralement sur le bras (2) de la pièce à usiner, à une distance prédéterminée de l'axe de la pièce à usiner (3) et en particulier également à une distance de la pièce à usiner (3).

5. Fraiseuse dentaire (1) selon l'une des revendications précédentes, **caractérisée en ce que** le dispositif de commande (18) permet de commander le mouvement relatif de l'élément d'appui (28) et de la pointe (14a) de l'outil (14) sous la forme d'un mouvement linéaire de translation vers l'outil (14) et à partir de celui-ci.

6. Fraiseuse dentaire (1) selon l'une des revendications précédentes, **caractérisée en ce que** le dispositif de commande (18) permet de commander le bras porte-pièce (2) avec l'élément d'appui (28) vers la pointe (14a) de l'outil (14) de telle sorte que l'élément d'appui (28) entre toujours en contact avec la pointe (14a) de l'outil (14) au même endroit, même lors du changement d'outil.

7. Fraiseuse dentaire (1) selon l'une des revendications précédentes, **caractérisée en ce que** la fraiseuse dentaire (1) est conçue comme une machine-outil 5/0, c'est-à-dire avec cinq axes de déplacement du bras porte-pièce (2).

8. Fraiseuse dentaire (1) selon la revendication 2, **caractérisée en ce que** le dispositif de commande (18) pour la détermination du point de référence commute l'encodeur (19) du moteur d'entraînement du bras porte-pièce (2) de la boucle ouverte à la boucle fermée.

9. Fraiseuse dentaire (1) selon la revendication 2, **caractérisée en ce que** la combinaison du dispositif de commande (18) et de l'encodeur (19) est conçue comme un capteur de glissement par lequel le dispositif de commande (18) constate ou signale l'appui de l'élément d'appui (28) sur la pointe de l'outil en cas d'apparition d'un glissement lors du mouvement du bras de la pièce à usiner (2) vers la pointe de l'outil.

10. Fraiseuse dentaire (1) selon l'une des revendications précédentes, **caractérisée en ce que** l'outil de fraisage (14) présente un code et qu'un dispositif de reconnaissance d'outil de fraisage (22) détecte l'outil de fraisage (14) de manière spécifique à l'outil à l'aide de ce code et que la détection est mémorisée dans le dispositif de commande (18).

11. Procédé de détermination de la position de la pointe (14a) d'un outil (14) d'une fraiseuse dentaire (1) pour la fixation d'un point de référence, où l'outil (14) est serré dans un mandrin (12) et un bras (2) de la pièce à usiner présente un élément d'appui (28), à un endroit qui est séparé de la pièce à usiner (3), notamment son axe, à une distance prédéterminée, **caractérisé en ce que** pour la détermination du point de référence, le bras (2) de la pièce à usiner avec l'élément d'appui (28) est déplacé en translation par rapport à la pointe (14a) de l'outil (14) par un moteur d'entraînement pour le bras (2) de la pièce à usiner, où le mouvement du bras (2) de la pièce à usiner est détecté par un encodeur (19) qui est couplé au moteur d'entraînement, et **en ce que** l'encodeur (19) agit conjointement avec le dispositif de commande (18) comme un capteur de glissement qui détecte lorsqu'un glissement d'entraînement se produit en raison de l'élément d'appui (28) sur la pointe (14a) de l'outil (14), et **en ce que** la première apparition d'un glissement d'entraînement est évaluée comme un signe de l'appui et donc par le dispositif de commande (18) comme une position de référence.

12. Procédé selon la revendication 11, **caractérisé en ce que** le bras de la pièce à usiner (2) est déplacé avec l'élément d'appui (28) vers la pointe (14a) de l'outil (14), qui est serré dans une broche d'outil montée de manière fixe, par l'intermédiaire d'un moteur d'entraînement conçu comme un moteur pas à pas, et une perte de pas du moteur pas à pas est utilisée pour la détection du glissement.
